# EUROPÄISCHE PATENTSCHRIFT

(11) **EP 0 556 681 B1**
(45) Veröffentlichungstag und Bekanntmachung des Hinweises auf die Patenterteilung: **28.05.1997**
(21) Anmeldenummer: 93101918.6
(22) Anmeldetag: 08.02.1993
(51) Int. Cl.: C07C 67/347, C07C 69/716

(54) **Verfahren zur Herstellung von omega-Formylalkancarbonsäureestern**
Process for the preparation of esters of omega-formylalkanoic acids
Procédé de préparation d'esters d'acides oméga-formylalcanecarboxyliques

(30) Priorität: 18.02.1992 DE 4204808
(43) Veröffentlichungstag der Anmeldung: 25.08.1993
(73) Patentinhaber: BASF Aktiengesellschaft, 67063 Ludwigshafen (DE)
(72) Erfinder: Roeper, Michael, Prof. Dr., W-6706 Wachenheim (DE); Lorz, Peter Michael, Dr., W-6800 Mannheim 1 (DE); Koeffer, Dieter, Dr., W-6940 Weinheim (DE)

(56) Entgegenhaltungen:
- EP-A- 0 125 567
- EP-A- 0 306 094
- US-A- 4 748 261
- US-A- 4 769 498
- Canadian Journal of Chemistry, 46, 1968, 2225; D. McGreer
- Bulletin of the Chemical Society of Japan, 46, 1973, 528; A. Matsuda
- Tetrahedron Letters, 28, 1972, 5769; H. Singer

## Beschreibung

Gegenstand der Erfindung ist ein Verfahren zur Herstellung von ω-Formylalkancarbonsäureestern durch Hydroformylierung von Alkencarbonsäureestern.

Aus der US-Patentschrift 3 253 018 und EP-A 295 554 ist bekannt, daß man ω-Formylalkancarbonsäureester durch Hydroformylieren von ω-Alkencarbonsäureestern in Gegenwart von Kobaltcarbonylkatalysatoren erhält. Hierbei entstehen jedoch erhebliche Mengen an verzweigten Estern, die für die weitere Verwendung unerwünscht sind.

In der EP-B-125 567 und EP-B 131 860 werden auch Verfahren beschrieben, bei denen man 3-Pentensäureester oder 4-Pentensäureester in Gegenwart von Carbonylkomplexen des Rhodiums, die mit tertiären, organischen Phosphinen oder Phosphiten modifiziert sind, hydroformyliert und Formylvaleriansäureester erhält. Der Anteil an geradkettigen Verbindungen ist jedoch noch verbesserungsbedürftig.

Die US 3,527,809 und US 4,599,206 beschreiben Triorganophosphin-, Triorganophosphit- und Diorganophosphitliganden als Bestandteile eines Metall-Phosphor-Ligand-Komplex-Katalysators, die zur Hydroformylierung eingesetzt werden können. Ausgehend von diesen Liganden sollten in der US 4,769,498 und in der US 4,748,261 effektivere und stabilere Ligandensysteme entwickelt werden. Als solche werden in den beiden letztgenannten US-Patenten tertiäre Polyphosphite als Liganden für die rhodiumkatalysierte Hydroformylierung empfohlen. Ein Hinweis, wie der Anteil an 5-Formylvaleriansäureestern bei der Hydroformylierung von 2- und 3-Pentensäurestern erhöht werden kann, wird in den US-Patenten nicht gegeben.

Es war deshalb die technische Aufgabe gestellt, ein Verfahren zur Herstellung von ω-Formylalkancarbonsäureestern durch Hydroformylierung von Alkencarbonsäureestern zur Verfügung zu stellen, bei dem man einen möglichst hohen Anteil an geradkettigen Verbindungen erhält, die Reaktion unter mäßigen Überdruck mit hohem Umsatz verläuft und die verwendeten Katalysatoren eine erhöhte Stabilität aufweisen.

Diese technische Aufgabe wird gelöst in einem Verfahren zur Herstellung von ω-Formyl-C4-C20-Alkancarbonsäureestern durch Umsetzen von C4-C20-Alkencarbonsäureestern mit Kohlenmonoxid und Wasserstoff bei einer Temperatur von 30 bis 150°C in flüssiger Phase in Gegenwart von Rhodiumcarbonylkomplexen und in Gegenwart von mindestens einem tertiären organischem Polyphosphit mit 2 bis 6 Phosphoratomen im Molekül, in dem jeweils eine Bindung an jedem Phosphoratom über eine Sauerstoffbrücke an einen substituierten oder nicht substituierten mindestens zweiwertigen Arylen- oder Bisarylenrest, einen Alkylenrest, der ein Sauerstoffatom in Kette enthalten kann, oder an einen Rest mit zwei isolierten Arylresten über die Arylreste gebunden ist und zwei Bindungen an jedem Phosphoratom über eine Sauerstoffbrücke an einen substituierten oder nicht substituierten zweiwertigen Arylen-, Bisarylenrest, einen Alkylenrest oder einen Rest mit zwei isolierten Arylresten über die Arylreste gebunden sind, oder zwei Bindungen an mindestens einem Phosphoratom jeweils getrennt über eine Sauerstoffbrücke jeweils an einen einwertigen substituierten oder nicht substituierten Aryl-, Bisaryl-, Alkyl-, Aralkyl- oder Cycloalkylrest gebunden sind, indem man als C4-C20-Alkencarbonsäureester 3- oder 2-Pentensäureester verwendet.

Das neue Verfahren hat den Vorteil, daß es mit größerer Selektivität zu geradkettigen Verbindungen führt, wobei bei mäßigem Überdruck ein hoher Umsatz erzielt wird und die verwendeten Rhodiumkatalysatoren eine erhöhte Stabilität aufweisen.

Als Ausgangsverbindung verwendet man erfindungsgemäß 3- oder 2-Pentensäureester als C₄-C₂₀-Alkencarbonsäureester, insbesondere deren C₁-C₄-Alkylester. Die olefinische Doppelbindung kann endständig oder intern sein. Bevorzugt geht man von C₄-C₇-Alkencarbonsäure-C1-C4-Alkylestern aus. Besondere technische Bedeutung haben 2-Pentensäure-C₁-C₄-Alkylester und 3-Pentensäure-C₁-C₄-Alkylester sowie deren Gemische mit 4-Pentensäure-C₁-C₄-Alkylester erlangt. Geeignet sind z.B. 4-Pentensäureethylester, 3-Pentensäurepropylester, 2-Pentensäureethylester sowie deren Gemische. Besonders bevorzugt sind 4-Pentensäuremethylester und 3-Pentensäuremethylester. Weiterhin bevorzugt sind Gemische aus 2-, 3- und 4-Pentensäureestern wie aus 4-, 3- und 2-Pentensäuremethylestern sowie 2-Pentensäuremethylester.

Die C₄-C₂₀-Alkencarbonsäureester werden mit Kohlenmonoxid und Wasserstoff umgesetzt. In der Regel enthält das Gasgemisch Wasserstoff:Kohlenmonoxid im Molverhältnis von 1:10 bis 100:1, insbesondere von 1:1 bis 40:1.

Die Hydroformylierung wird bei einer Temperatur von 30 bis 150°C in flüssiger Phase durchgeführt. Vorteilhaft wendet man eine Temperatur von 50 bis 120°C an. In der Regel führt man die Umsetzung unter einem Druck von 0,01 bis 30 bar vorteilhaft 1 bis 30 bar durch. Besonders bewährt haben sich Drücke von 1 bis 20 bar.

Vorteilhaft wird die Umsetzung in Gegenwart von Lösungsmitteln durchgeführt. Geeignete Lösungsmittel sind beispielsweise aromatische Kohlenwasserstoffe, wie Toluol oder Xylol, ferner die bei der Hydroformylierung jeweils anfallenden ω-Formylalkancarbonsäureester oder bei der Hydroformylierung anfallende Hochsieder.

Die Hydroformylierung wird in Gegenwart von Rhodiumcarbonylkomplexen und mindestens einem tertiären organischen Polyphosphit mit 2 bis 6 Phosphoratomen durchgeführt. Die Konzentration des Rhodiumkomplexkatalysators beträgt im allgemeinen von 10 bis 1000 ppm, berechnet als Rhodiummetall, vorzugsweise 10 bis 500 ppm Rhodium und insbesondere 25 bis 350 ppm Rhodium.

Im allgemeinen werden 2 bis 100, vorzugsweise 3 bis 50 Mol Polyphosphit pro Gramm Atom Rhodium (das ist die Summe aus dem komplexierten und freien Polyphosphit) eingesetzt. Frisches Polyphosphit kann zu jedem Zeitpunkt der Reaktion zugesetzt werden, um die Konzentration an freiem, nicht komplexierten Phosphit konstant zu halten. Der Rhodiumcarbonylpolyphosphitkomplex-Katalysator kann vor der Verwendung getrennt hergestellt werden. In der Regel werden aber die katalytisch aktiven Komplexe aus einem Katalysatorvorläufer, wie Rhodiumcarbonylacetylacetonat, Rhodiumoxid, Rhodiumcarbonyl, Rhodiumnitrat oder Rhodiumacetat und dem Polyphosphitliganden im Reaktionsmedium gebildet. Vorzugsweise werden Rhodiumcarbonylacetylacetonat oder Rhodiumacetat als Rhodiumkomponente eingesetzt, die in Gegenwart eines Lösungsmittel mit dem Polyphosphitliganden umgesetzt wird, um den Vorläufer des katalytisch aktiven Komplexes zu bilden, der in der Reaktion zusammen mit überschüssigem Polyphosphit eingebracht wird, um dort unter Reaktionsbedingungen in situ den aktiven, modifizierten Rhodiumcarbonylkomplex zu bilden.

Bevorzugte tertiäre organische Phosphite haben 2 bis 4 insbesondere 2 Phosphoratome. Jeweils eine Bindung an jedem Phosphoratom ist über eine Sauerstoffbrücke an einen mindestens zweiwertigen z.B. zwei bis vier wertigen Arylen- oder Bisarylenrest mit bis zu 12 Kohlenstoffatomen, einen Alkylenrest mit 2 bis 8 Kohlenstoffatomen, der ein Sauerstoffatom in der Kette enthalten kann, oder einen Rest mit bis 16 Kohlenstoffatomen mit zwei isolierten Arylresten z.B. 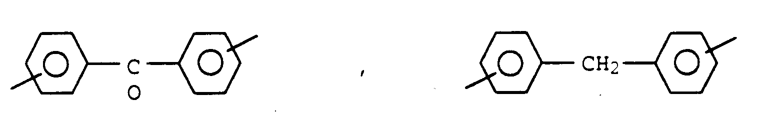 gebunden.

Die zwei restlichen Bindungen an jedem Phosphoratom sind jeweils über eine Sauerstoffbrücke an einen zweiwertigen Arylen- oder Bisarylenrest mit bis zu 20 Kohlenstoffatomen, einen Alkylenrest mit 4 bis 8 kohlenstoffatomen oder an einen Rest mit zwei isolierten Arylresten, wie oben definiert, über die Arylreste gebunden. Die Aryl- und Bisarylreste haben vorteilhaft in o und p Stellung C₁-C₄-Alkoxygruppen, insbesondere Methoxygruppen, ferner C₁-C₄-Alkylgruppen, insbesondere t-Butylgruppen, als Substituenten.

Bevorzugte Polyphosphite sind Verbindungen der Formel I 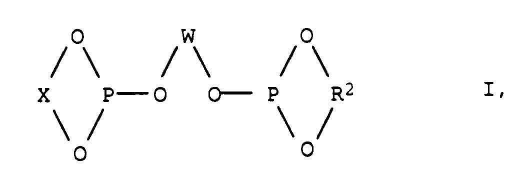 in der
- X: einen zweiwertigen Bisarylenrest oder R¹,
- W: einen zweiwertigen substituierten oder nicht substituierten Arylen-, Bisarylen- oder Alkylenrest und
- R¹ und R²: gleich oder verschieden sind und einen substituierten oder unsubstituierten Allen oder ortho-Arylenrest bedeuten.

Von den Verbindungen der Formel I sind solche bevorzugt, bei denen die Reste X und W in der Formel I Bisarylenreste, insbesondere den Rest der Formel II 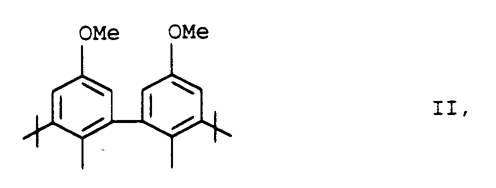 und R² einen ortho-Phenylen-, 2,2-dimethylpropylen-1,3- oder 1,1,2,2-Tetramethylethylenrest bedeuten. Ferner sind solche Verbindungen der Formel I hervorzuheben, bei denen W, R¹ und R² unabhängig voneinander ortho-Phenylen-, 2,2-Dimethylpropylen-1,3- oder 1,1,2,2-Tetramethylethylenreste bedeuten.

Die Polyphosphite der Formel I können nach an sich bekannten Methoden durch eine geeignet gewählte Abfolge von Phosphorhalogenid-Alkohol-Kondensationsreaktion hergestellt werden.
a) Beispielsweise setzt man Phosphortrichlorid mit einem Diol unter Bildung eines Monochlorphosphites um;
b) setzt dieses Zwischenprodukt mit einem weiteren Diol unter Bildung des korrespondierenden hydroxylsübstituierten Diorganophosphites um;
c) läßt dieses Diorganophosphitzwischenprodukt mit Phosphortrichlorid unter Bildung des korrespondierenden Phosphordichloridzwischenproduktes reagieren;
d) und schließt mit der Reaktion dieses Dichlorids mit einem entsprechenden Diol unter Bildung des gewünschten Bisphosphites ab.

Während dieser Syntheseweg für die Herstellung von unsymmetrisch substituierten Phosphiten erforderlich ist, lassen sich symmetrisch substituierte Verbindungen durch Reaktion des Produktes von Schritt a) mit einem entsprechenden Diol im Molverhältnis 2:1 herstellen.

Die genannten Kondensationsreaktionen werden in der Regel in einem geeigneten Lösungsmittel, z.B. Toluol in Gegenwart einer Hilfsbase, wie Triethylamin, als HCl-Akzeptor durchgeführt.

Geeignete Verbindungen der Formel I sind z.B.

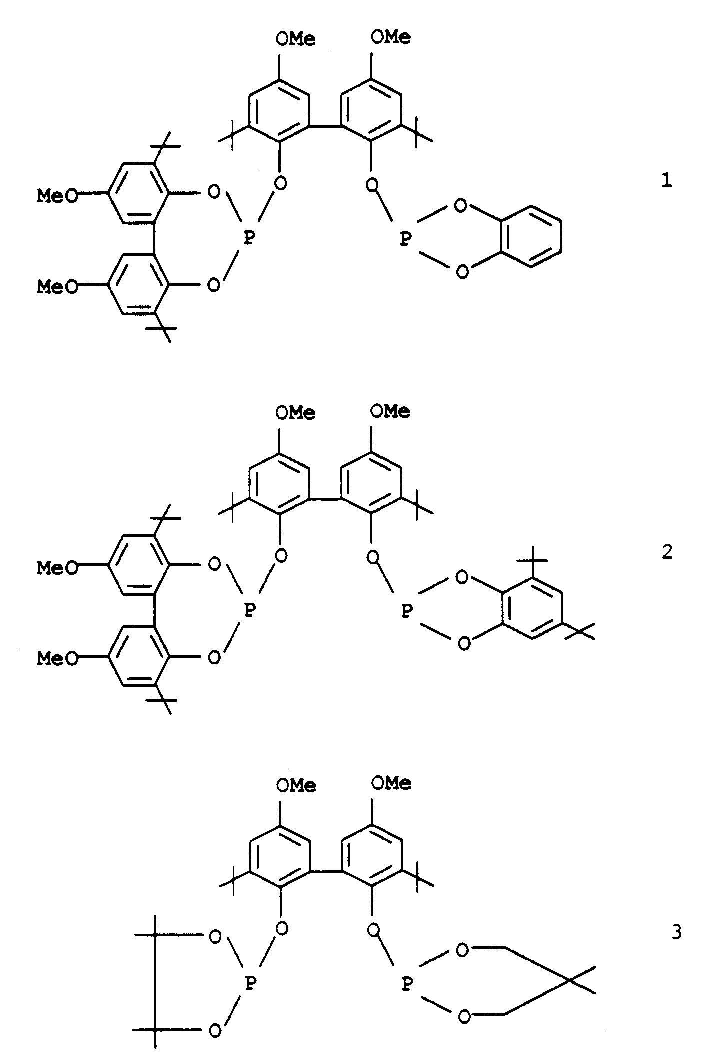

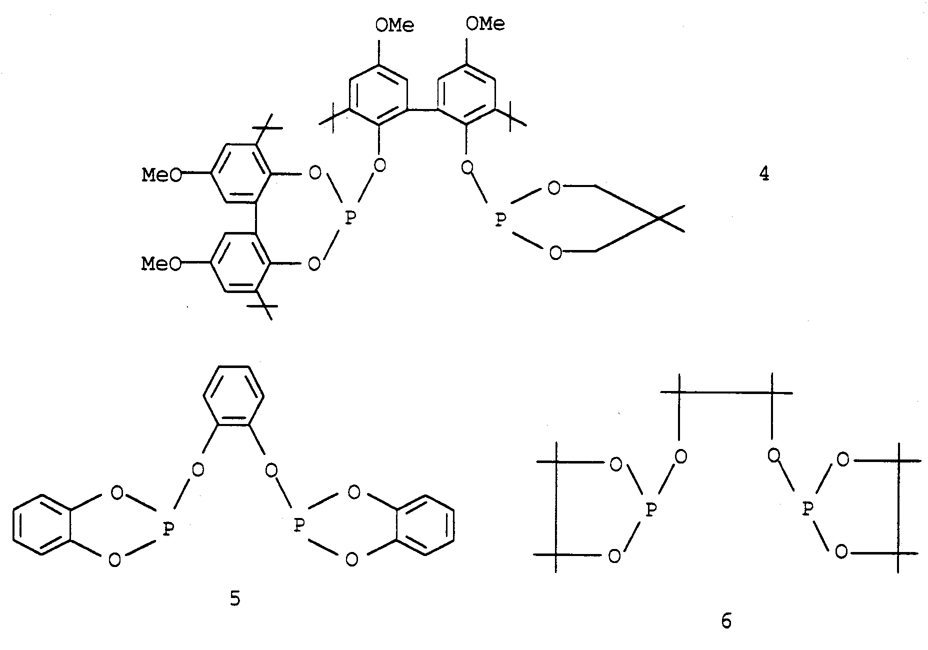

Eine weitere Klasse von geeigneten Polyphosphiten sind solche der Formel III

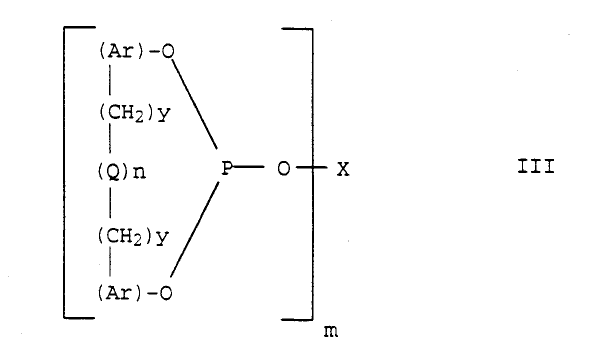

In der Formel III bedeuten
- Ar: jeweils ein Arylenradikal mit 6 bis 18 Kohlenstoffatomen, das gleich oder verschieden sein kann sowie substituiert oder nicht-sübstituiert sein kann;
- X: ein m-bindiges Radikal mit 2 bis 30 Kohlenstoffatomen, ausgewählt aus der Gruppe Alkylen, Alkylen-oxy-alkylen, ein Arylen oder ein Rest der Formel

Arylen-(CH₂)_{y}-(Q)ₙ-(CH₂)_{y}-Arylen

in der Arylenradikale wie oben definiert sind und
- y: 0 oder 1 ist;
- Q: eine bivalente Brückengruppe, ausgewählt aus Sauerstoff, Schwefel, -CO-, -CR¹R²- bedeutet, in der R¹ und R² jeweils ein Wasserstoffatom oder ein Alkylrest mit 1 bis 12 Kohlenstoffatomen oder einen Phenyl-, Tolyl- oder Anisylrest bezeichnen und -NR³-, in der R3 ein Wasserstoffatom oder einen Methylrest bezeichnet;
- n: 0 oder 1 bedeutet und
- m: eine ganze Zahl von 2 bis 6 bezeichnet.

In bevorzugten Verbindungen der Formel III bezeichnen Ar jeweils einen Phenylenrest, y und n 0 und m 2, wobei die beiden Phenylenreste in o-Stellung miteinander verbunden sind und in o- und p-Stellung zur Bindung an die Sauerstoffbrücke zum Phosphoratom Alkylgruppen mit 1 bis 4 Kohlenstoffatomen oder C₁-C₄-Alkoxygruppen insbesondere Methoxy- und t-Butylgruppen als Substituenten haben können.

Besondere technische Bedeutung hat ein Phosphit der Formel IV erlangt.

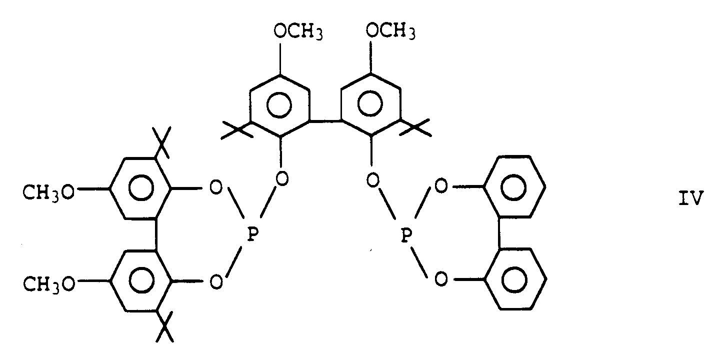

Weitere geeignete Verbindungen sind z.B. solche der Formeln V bis XXIX.

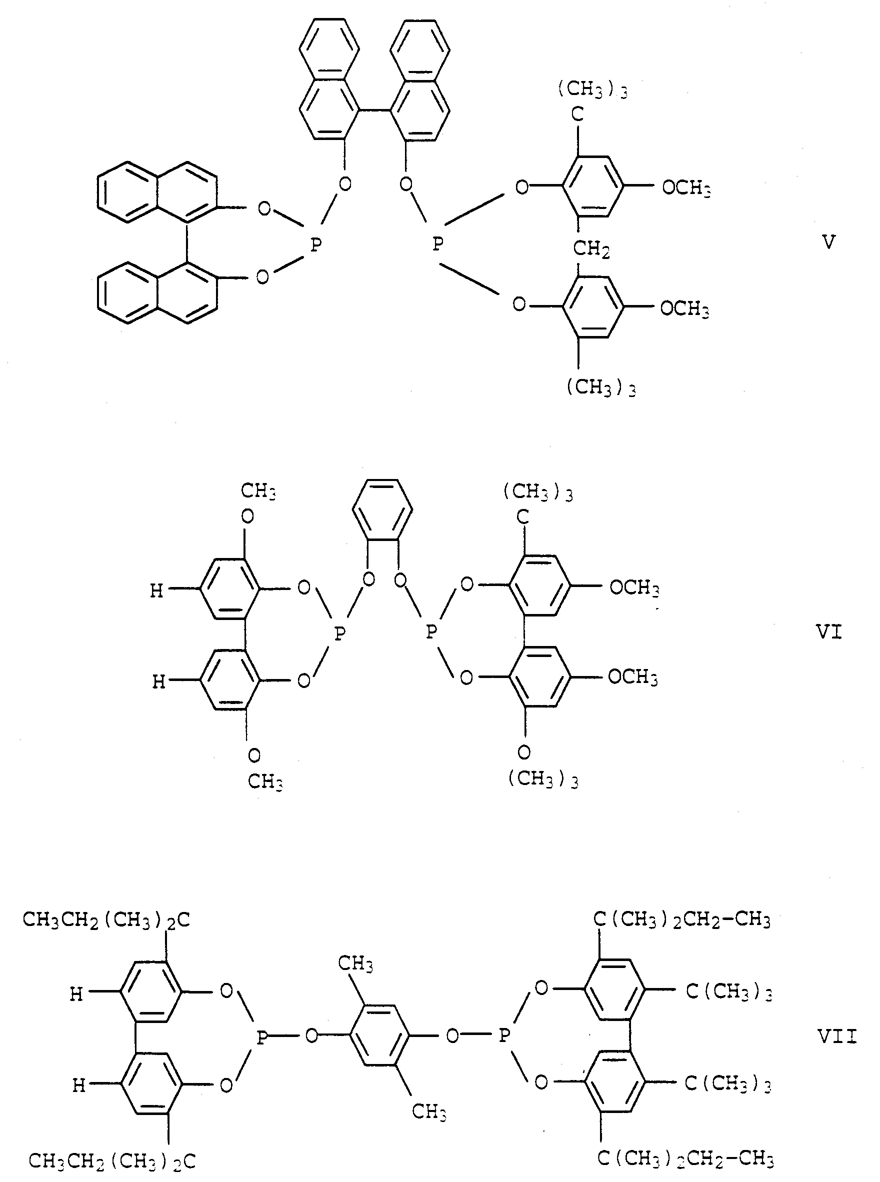

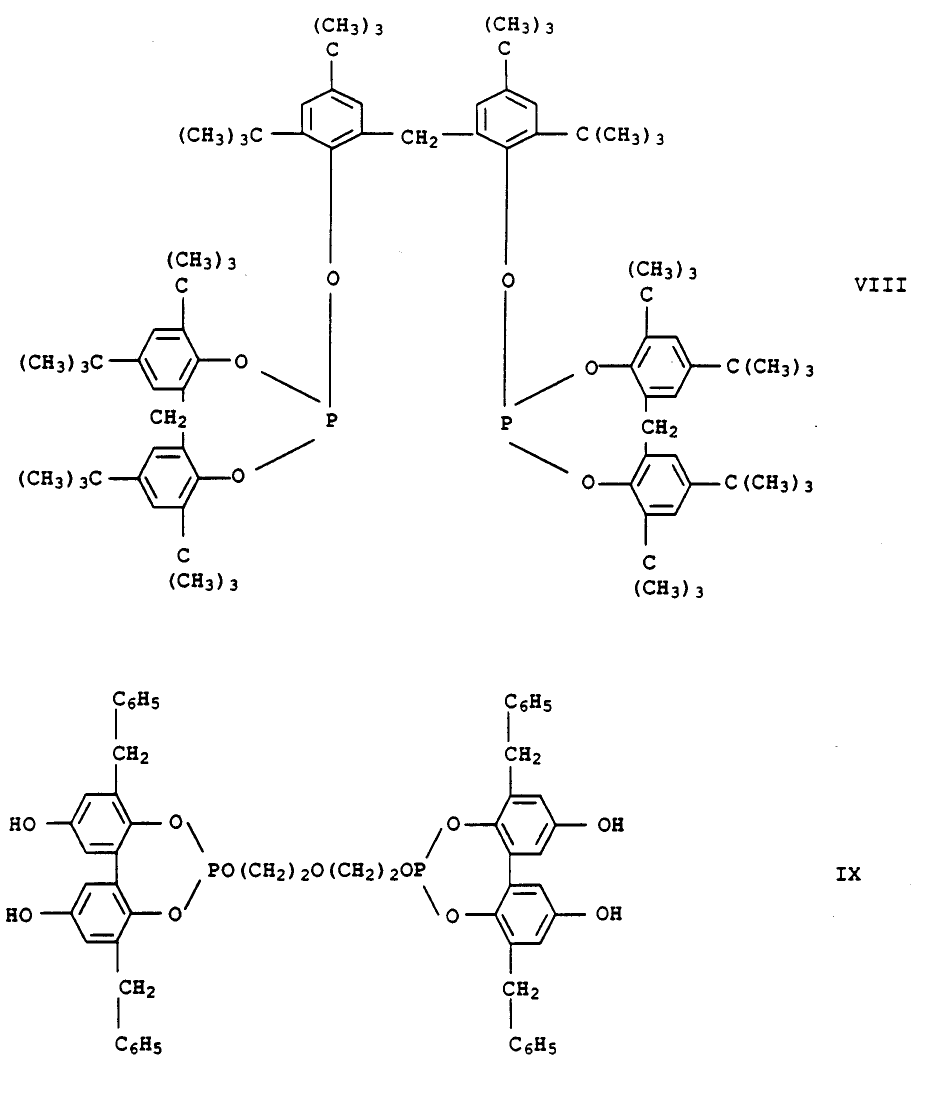

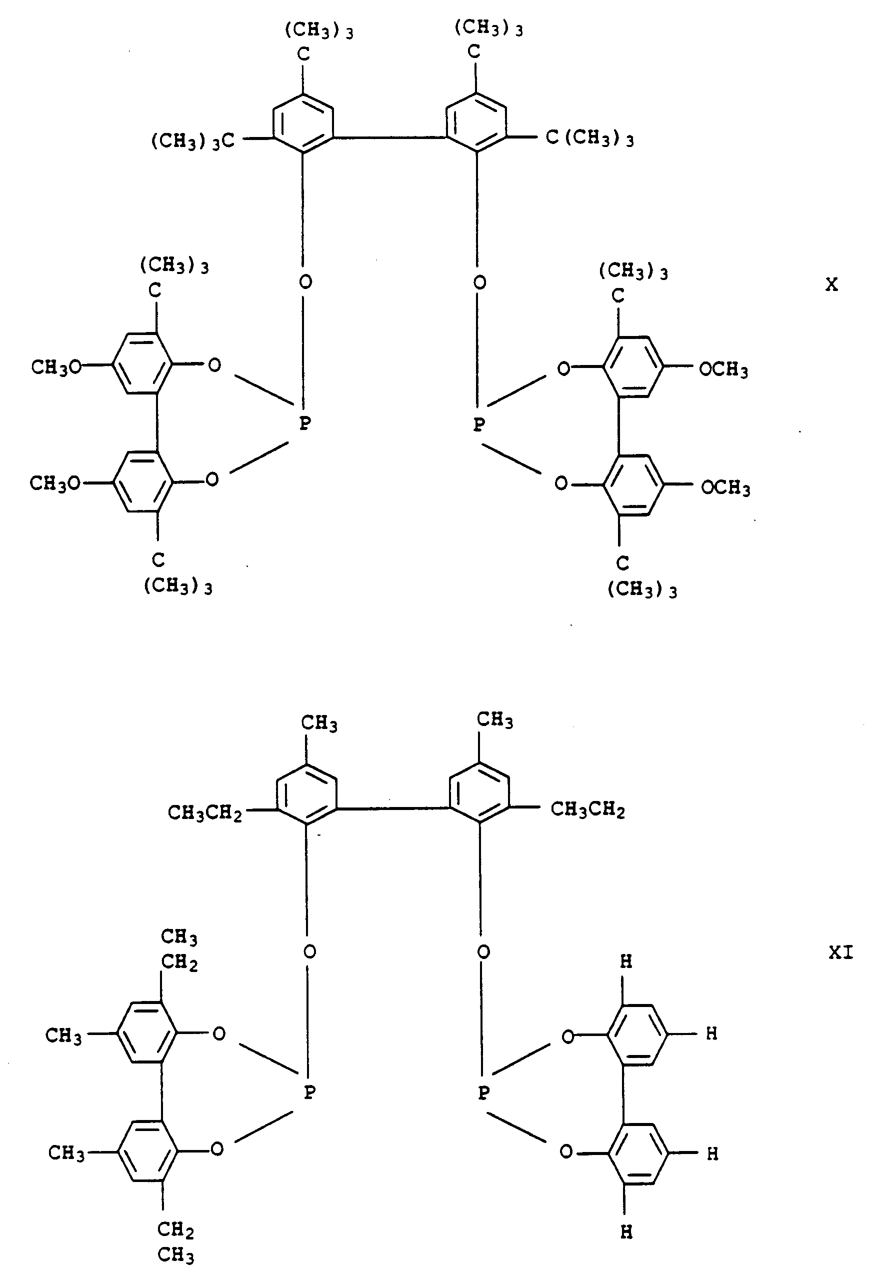

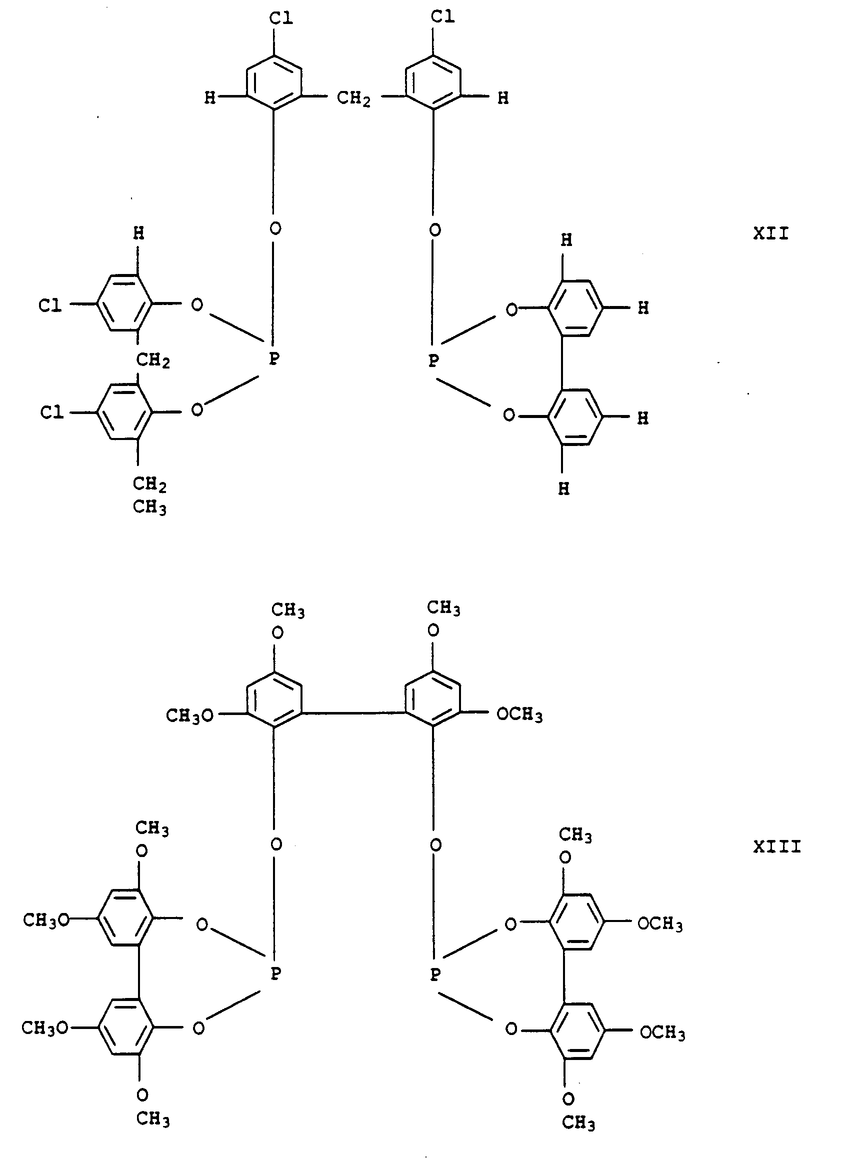

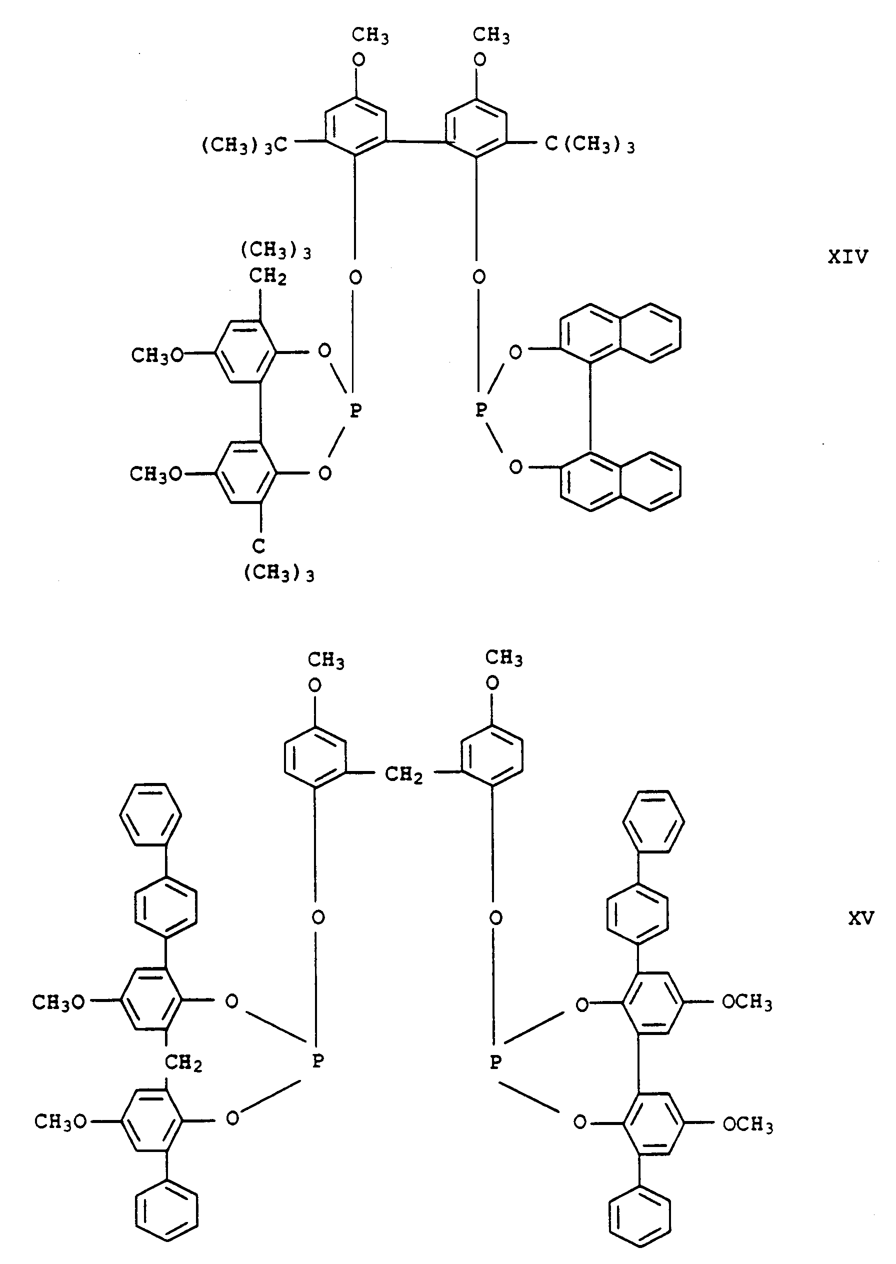

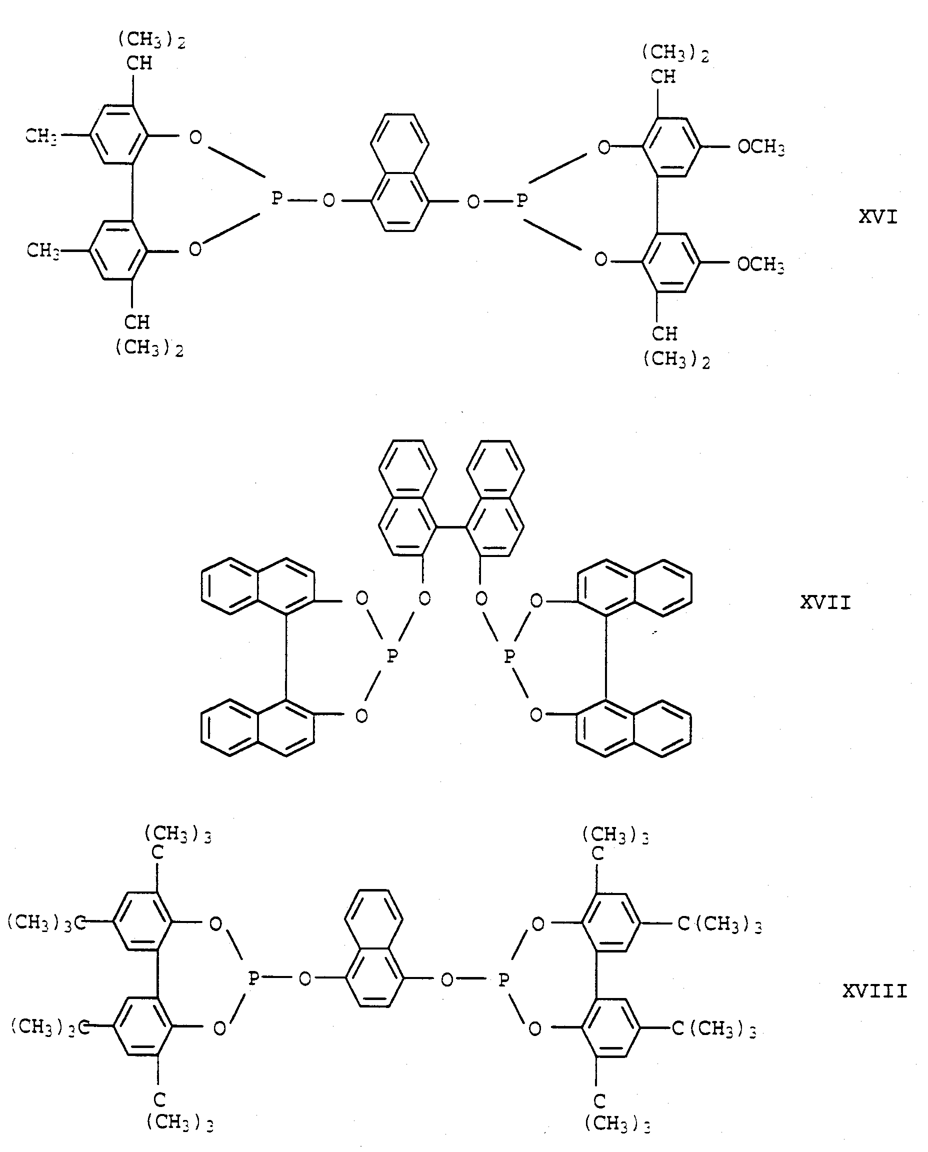

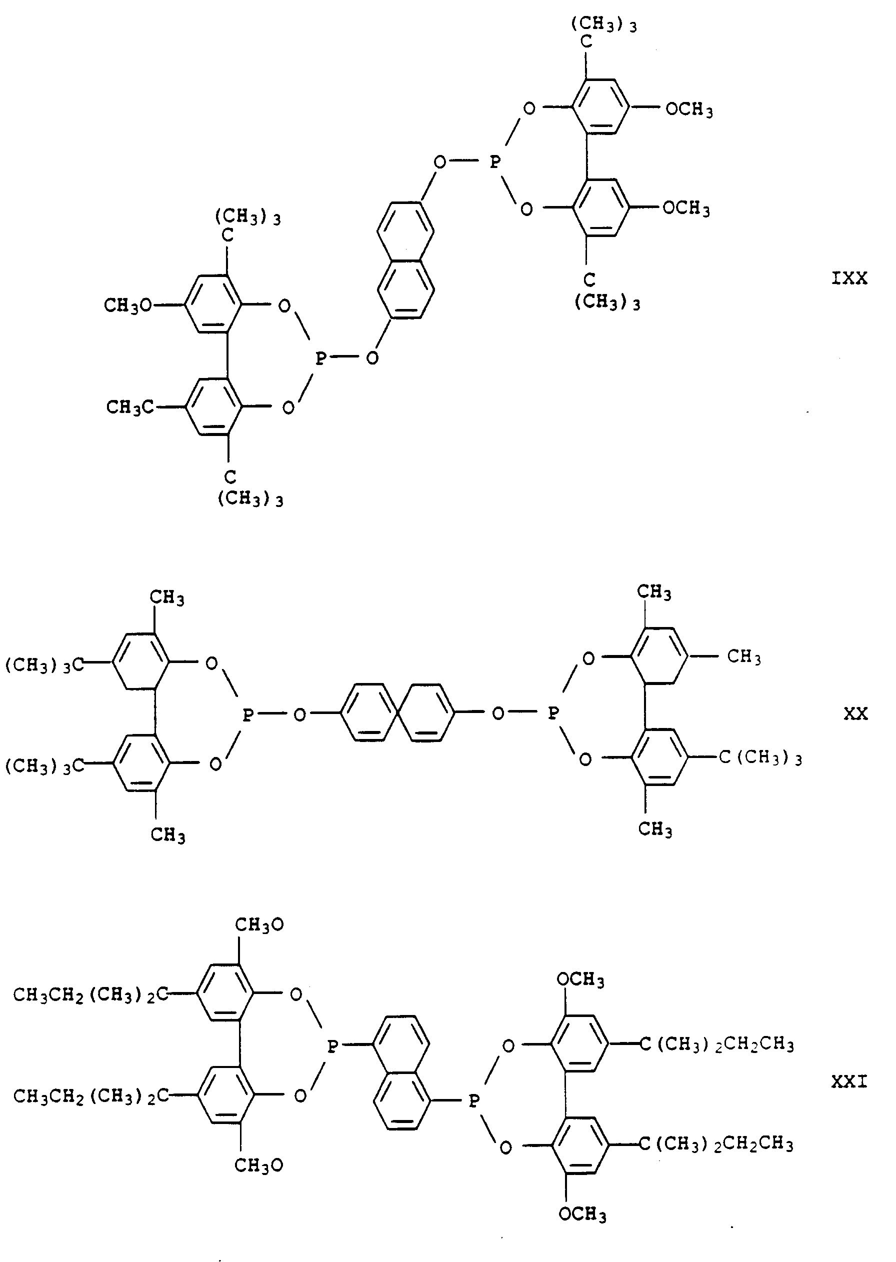

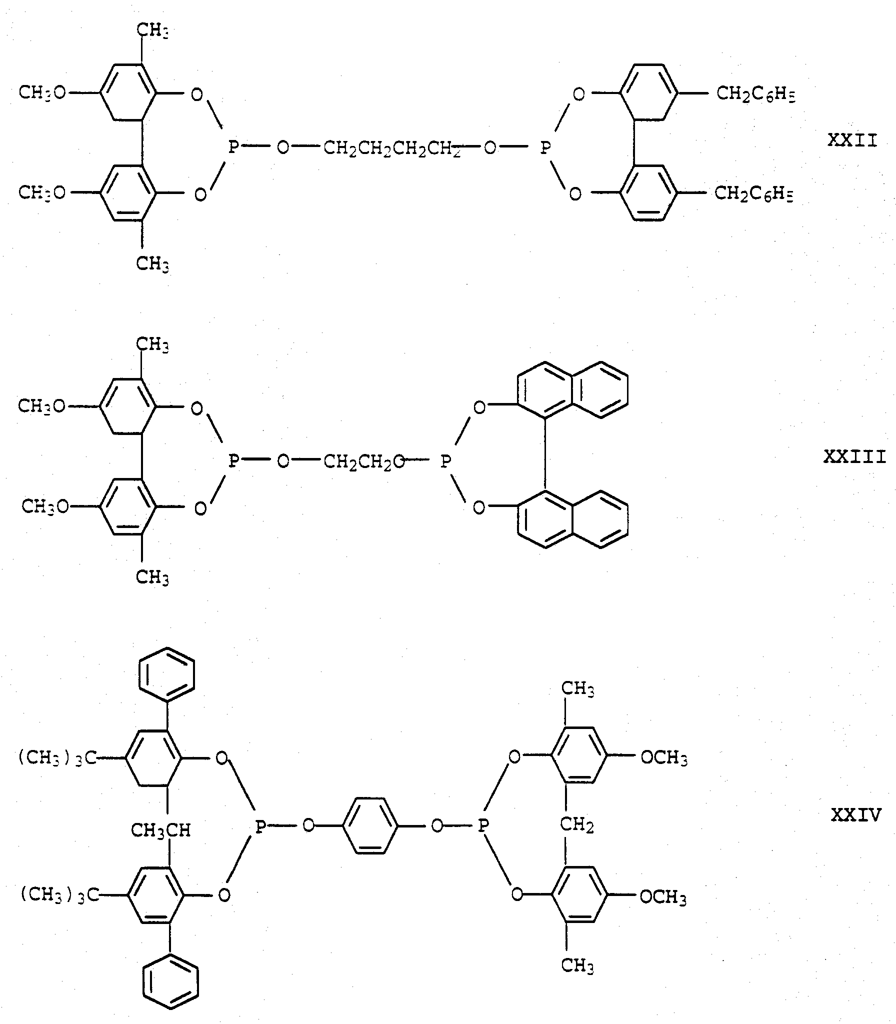

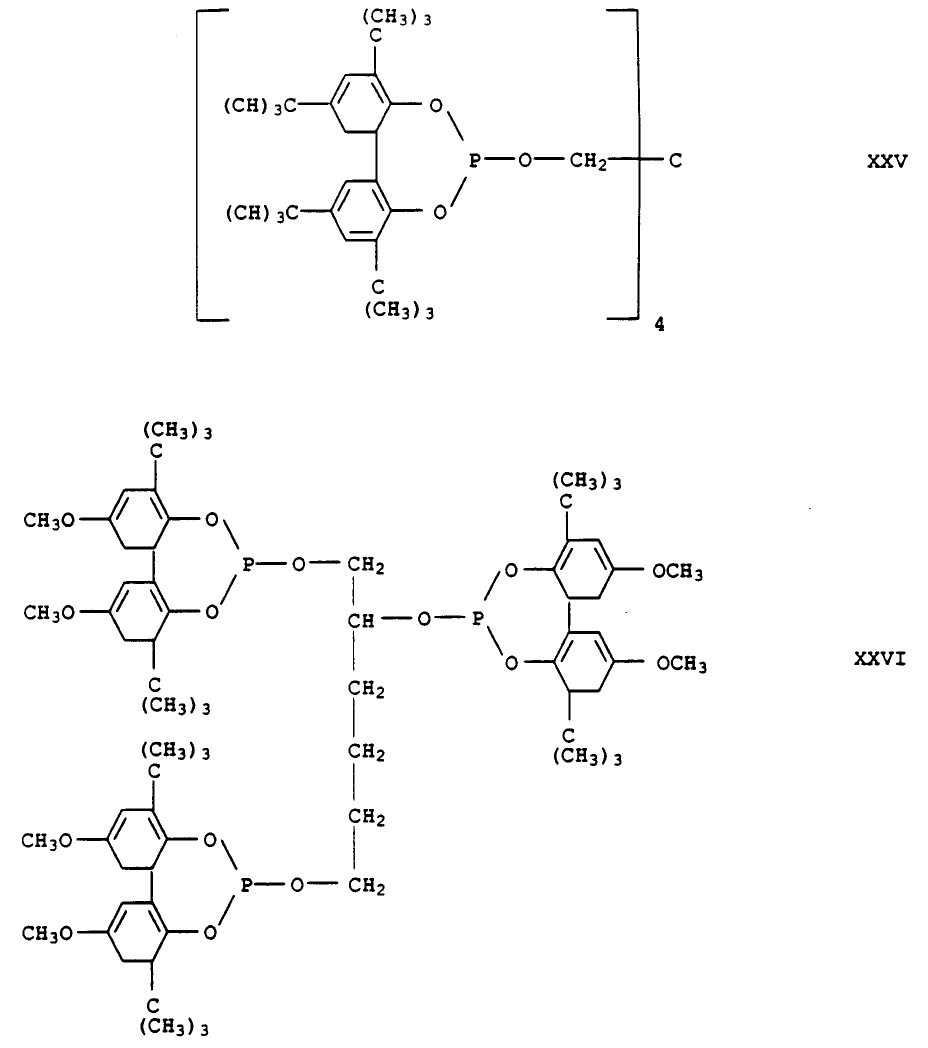

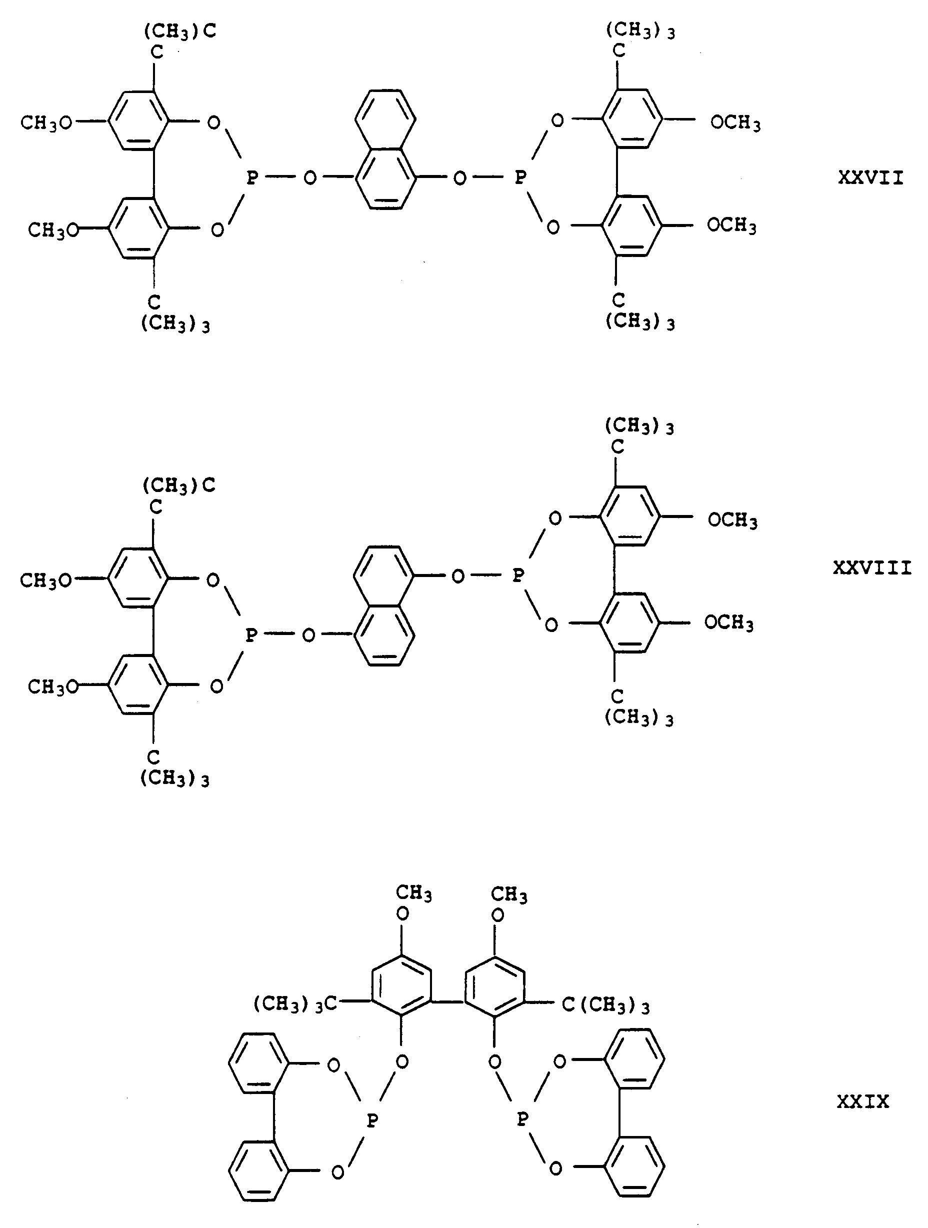

In besonders bevorzugten Verbindungen der Formel III bezeichnen
- Ar: jeweils einen Phenylenrest, der eine Methoxygruppe und/oder einen C₁- bis C₄-Alkylrest als Substituenten haben kann
- Y: 0
- n: 0 und
- m: 2

Eine weitere Klasse von bevorzugten Polyphosphiten sind solche der Formel XXX

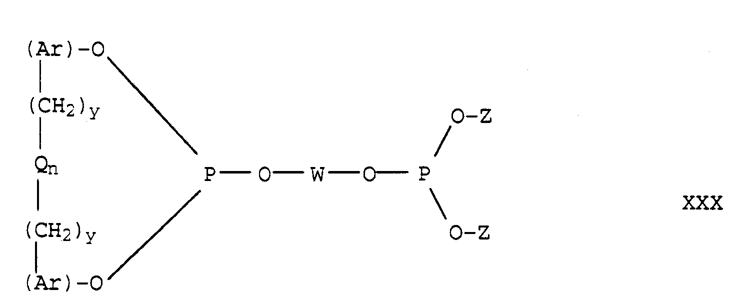

In der Formel XXX bedeuten
- Ar: jeweils ein Arylenradikal mit 6 bis 18 Kohlenstoffatomen, das gleich oder verschieden sein kann sowie substituiert oder nicht-substituiert sein kann;
- y: 0 oder 1;
- Q: eine bivalente Brückengruppe, ausgewählt aus Sauerstoff, Schwefel, -CO-, -CR¹R²-, in der R¹ und R² jeweils ein Wasserstoffatom oder ein Alkylrest mit 1 bis 12 Kohlenstoffatomen oder einen Phenyl-, Tolyl- oder Anisylrest bezeichnen und -NR³-, in der R³ ein Wasserstoffatom oder einen Methylrest bezeichnet;
- n: 0 oder 1;
- W: einen zweiwertigen sübstituierten oder nicht sübstituierten Arylen-, Bisarylen- oder Alkylenrest und
- Z: jeweils einen Alkyl-, Aryl-, Bisaryl-, Aralkyl- oder Cycloalkylrest, wobei Z gleich oder verschieden sein können.

In bevorzugten Verbindungen der Formel XXX bezeichnen Ar jeweils einen Phenylenrest, y und n 0 und m 2, wobei die beiden Phenylenreste in o-Stellung miteinander verbunden sind und in o- und p-Stellung zur Bindung an die Sauerstoffbrücke zum Phosphoratom Alkylgruppen mit 1 bis 10 Kohlenstoffatomen oder C₁-C₄-Alkoxygruppen insbesondere Methoxy- und t-Butylgruppen als Substituenten haben können.

W hat die vorgenannte Bedeutung und Z können gleich oder verschieden sein und bezeichnen jeweils einen Alkylrest mit 1 bis 18, insbesondere 1 bis 10 Kohlenstoffatomen, einen Aryl-, Bisaryl- oder Aralkylrest mit 6 bis 18 oder einen Cycloalkylrest mit 5 bis 8 Kohlenstoffatomen im Ring.

Geeignete Verbindungen sind beispielsweise diejenigen der Formeln

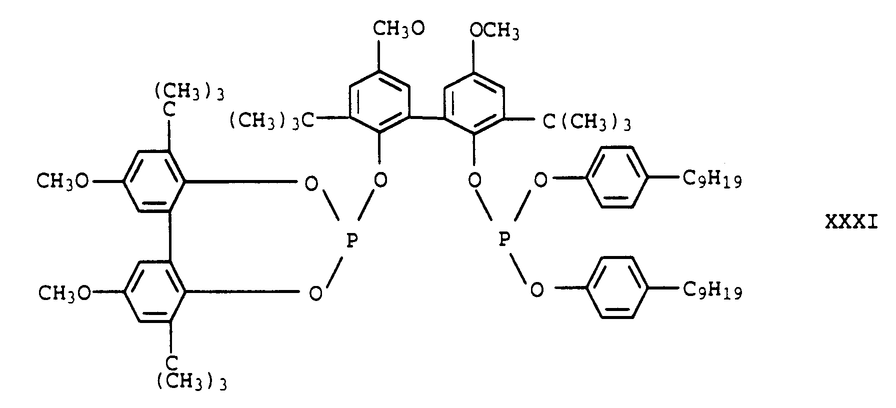

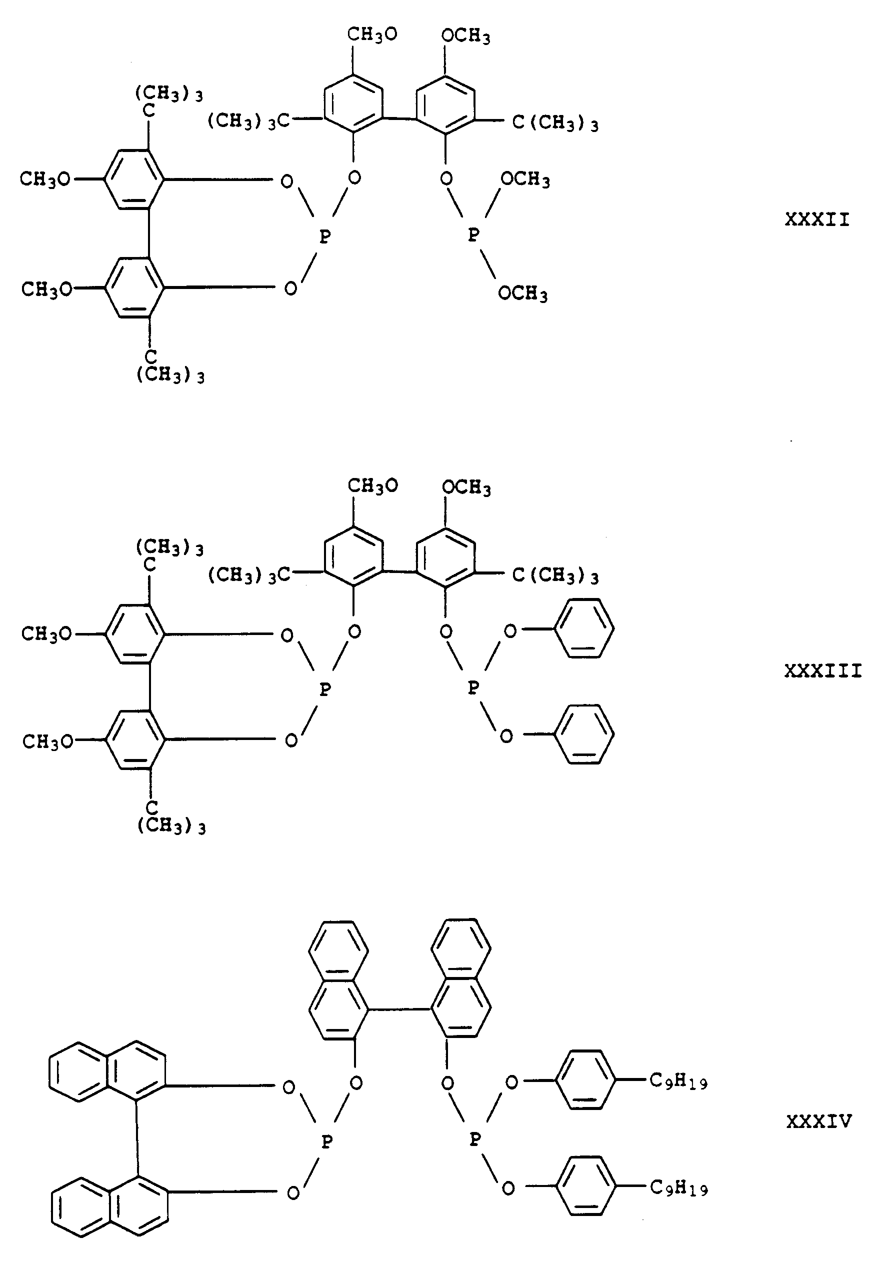

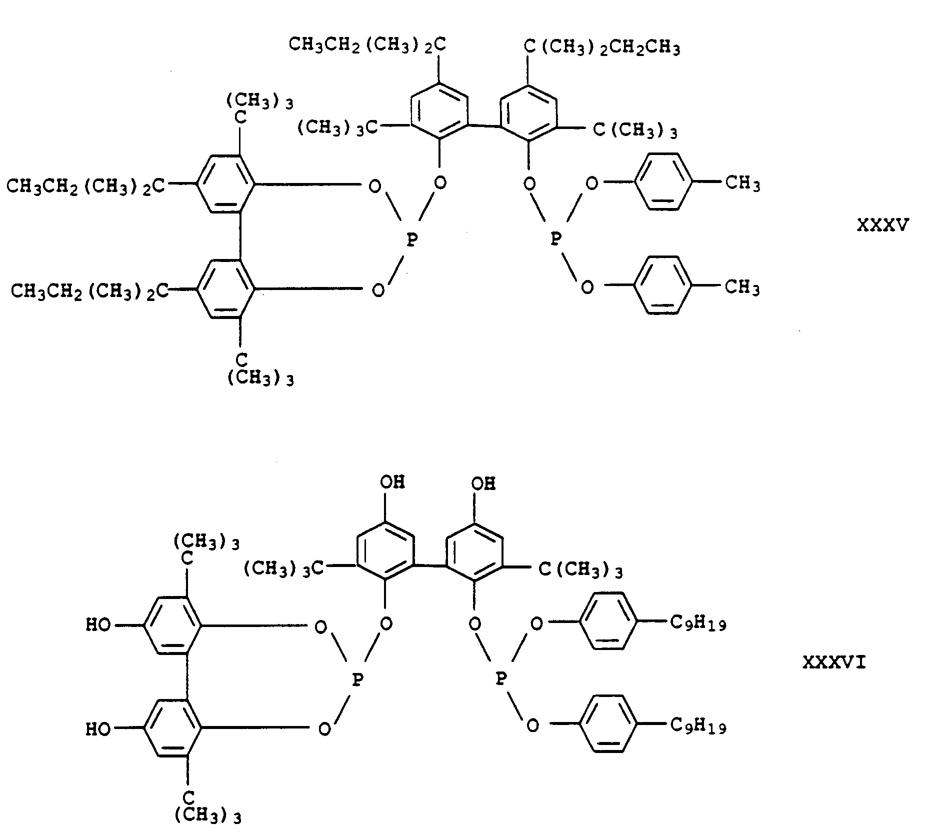

Verbindungen der Formel XXXI sind besonders bevorzugt.

Die erzeugten omega-Formylalkancarbonsäureester lassen sich nach bekannten Methoden, z.B. Destillation aus dem Reaktionsgemisch, abtrennen.

Die nach dem Verfahren der Erfindung erhältlichen omega-Formylalkancarbonsäureester eignen sich zur Herstellung von omega-Aminocarbonsäuren, die Ausgangsstoffe für Polymere sind.

Das Verfahren nach der Erfindung sei an folgendem Beispielen veranschaulicht.

### Beispiel 1

In einem 1 l-Hubrührautoklaven werden 100 g 3-Pentensäuremethylester in 300 g Toluol als Lösungsmittel der Hydroformylierung unterworfen. Das Reaktionsgemisch enthält als Katalysator 2,23 g der Verbindung IV (2,33 mmol) und 40 mg (0,388 mmol) Rhodium in Form der Komplexverbindung Rh(CO)₂acac (acac = Acetylacetonat). Das Reaktionsgemisch wird auf 100°C aufgeheizt und dann mit einem Gemisch aus 50 Vol.-% CO und 50 Vol.-% H₂ ein Druck von 5 bar eingestellt. Während der Reaktion wird der Druck im Reaktor durch Nachpressen eines Gasgemisches gleicher Zusammensetzung über einen Druckregler auf 5 bar gehalten. Nach einer Reaktionszeit von 5 Stunden ergibt die Analyse des Reaktionsgemisches folgende Umwandlung des 3-Pentensäuremethylester (in mol-%):

| | |
|---|---|
| Umsatz: | 95,5 mol-% |

| Selektivität | |
|---|---|
| 4-Pentensäuremethylester | 0,2 mol-% |
| 2-cis-Pentensäuremethylester | 0,2 mol-% |
| 2-trans-Pentensäuremethylester | 4,2 mol-% |
| Valeriansäuremethylester | 4,8 mol-% |
| 5-Formylvaleriansäuremethylester | 76,7 mol-% |
| 4-Formylvaleriansäuremethylester | 7,8 mol-% |
| 3-Formylvaleriansäuremethylester | 6,1 mol-% |

Die Ausbeute an Wertprodukt 5-Formylvaleriansäuremethylester beträgt 73,2 % und das Verhältnis 5-Formylvaleriansäuremethylester zu 3- und 4-Formylvaleriansäuremethylester beträgt 85:15.

Die Selektivität zu 5-Formylvaleriansäuremethylester ist mit 76,7 % gegenüber dem Stand der Technik 68,7 % (Hydroformylierung mit Cobalt) deutlich verbessert. Noch deutlicher wurde die Verbesserung beim Vergleich der Ausbeuten (73,2 % gegenüber 22,3 %) und des Verhältnisses von 5-Formylvaleriansäuremethylester zu 3- und 4-Formylvaleriansäuremethylester (85:15 gegenüber 69:31). Darüber hinaus wird dieses Ergebnis bei deutlich niedrigerem Druck erzielt.

Die direkte Hydroformylierung von 3-Pentensäuremethylester zu 5-Formylvaleriansäuremethylester gelingt mit konventionellen Rhodium/Triphenylphoshinkatalysatoren nur mit sehr schlechten Selektivitäten, wie aus dem Vergleichsbeispiel 1 deutlich wird.

### Vergleichsbeispiel 1

In einem 1 l-Hubrührautoklaven werden 100g 3-Pentensäuremethylester in 300 g Toluol als Lösungsmittel der Hydroformylierung unterworfen. Das Reaktionsgemisch enthält als Katalysator 26,2 g Triphenylphosphin (100 mmol) und 40 mg (0,388 mmol) Rhodium in Form der Komplexverbindung Rh(CO)₂acac (acac = Acetylacetonat). Das Reaktionsgemisch wird auf 100°C aufgeheizt und dann mit einem Gemisch aus 50 Vol.-% CO und 50 Vol.-% H₂ ein Druck von 5 bar eingestellt. Während der Reaktion wird der Druck im Reaktor durch Nachpressen eines Gasgemisches gleicher Zusammensetzung über einen Druckregler auf 5 bar gehalten. Nach einer Reaktionszeit von 5 Stunden ergibt die Analyse des Reaktionsgemisches folgende Umwandlung des 3-Pentensäuremethylester (in mol-%):

| | |
|---|---|
| Umsatz: | 34,0 mol-% |

| Selektivität | |
|---|---|
| 4-Pentensäuremethylester | 0,6 mol-% |
| 2-cis-Pentensäuremethylester | 0,0 mol-% |
| 2-trans-Pentensäuremethylester | 1,1 mol-% |
| Valeriansäuremethylester | 7,3 mol-% |
| 5-Formylvaleriansäuremethylester | 8,8 mol-% |
| 4-Formylvaleriansäuremethylester | 28,0 mol-% |
| 3-Formylvaleriansäuremethylester | 54,2 mol-% |

Die Ausbeute an Wertprodukt 5-Formylvaleriansäuremethylester beträgt nur 3,0 % und das Verhältnis 5-Formylvaleriansäuremethylester zu 3- und 4-Formylvaleriansäuremethylester beträgt 10:90. Eine wirtschaftliche Herstellung von 5-Formylvaleriansäuremethylester ist daher nach dem Verfahren des Vergleichsbeispiels 1 nicht möglich.

Im folgenden wird anhand einiger Beispiele der Anwendungsbereich des erfindungsgemäßen Verfahrens illustriert. Diese Beispiele sind jedoch keineswegs als Einschränkung aufzufassen, da gute Ergebnisse auch außerhalb des beschriebenen Bereichs erwartet werden können.

Der Einfluß von Druck und Temperatur kann den Beispielen 2-9 entnommen werden:

Beispiel 1 wurde wiederholt, wobei abweichend 1,49 g der Verbindung IV (1,55 mmol) eingesetzt wurden und die in Tabelle 1 genannten Drücke und Temperaturen eingestellt wurden. Der drucklose Versuch wurde in einem gerührten (Begasungsrührer mit Hohlwelle) Glaskolben durchgeführt, wobei über ein Gaseinleitungsrohr ein Gemisch aus 50 Vol.-% CO und 50 Vol.-% H₂ so zugeführt wurde, daß sich ein Abgasstrom von ca. 1 Blase/Sekunde (Blasenzähler) einstellte.

**Tabelle 1**

| Bsp. Nr. | Druck^{a} bar | Temp. °C | Umsatz % | Selektivität, mol-% | | | | | | 5-FVSE |
|---|---|---|---|---|---|---|---|---|---|---|
| | | | | 4-PSE | 2-PSE | VSE | 5-FVSE | 4-FVSE | 3-FVSE | FVSE |
| 2 | 4 | 90 | 61,9 | 2,2 | 12,5 | 2,8 | 66,6 | 9,1 | 6,8 | 81 |
| 3 | 4 | 100 | 84,7 | 0,7 | 13,1 | 6,4 | 68,4 | 7,4 | 4,1 | 86 |
| 4 | 4 | 110 | 60,9 | 5,1 | 38,8 | 4,9 | 43,8 | 4,9 | 2,6 | 85 |
| 5 | 0 | 90 | 63,1 | 3,8 | 22,1 | 9,5 | 55,6 | 7,3 | 1,7 | 86 |
| 6 | 0 | 100 | 67,5 | 3,9 | 52,3 | 16,3 | 22,6 | 4,2 | 0,8 | 82 |
| 7 | 6 | 100 | 91,2 | 0,0 | 15,4 | 5,9 | 59,5 | 11,9 | 7,4 | 76 |
| 8 | 8 | 100 | 81,7 | 0,6 | 20,3 | 4,8 | 54,9 | 11.4 | 7,9 | 74 |
| 9 | 10 | 110 | 94,7 | 0,0 | 14,6 | 9,3 | 55,8 | 12,7 | 7,6 | 73 |

| | | | | | | | | | | |
|---|---|---|---|---|---|---|---|---|---|---|
| ^{a}) Überblick | | | | | | | | | | |
| FVSE: Formylvaleriansäureester PSE: Pentensäureester | | | | | | | | | | |

Man erkennt, daß die Hydroformylierung bereits bei Normaldruck gelingt, bessere Ausbeuten jedoch im Druckbereich von 4-6 bar (Überdruck) erzielt werden. Der n-Anteil, das heißt das Verhältnis 5-Formylvaleriansäuremethylester zu 3- und 4-Formylvaleriansäuremethylester, fällt bei weiterer Erhöhung des Drucks ab.

Der Einfluß des Ligand/Rhodiumverhältnisses ist dem Beispiel 3 aus Tabelle 1 und den Beispielen 10 - 12 zu entnehmen:

Beispiel 1 wurde wiederholt, wobei abweichend die in Tabelle 2 genannten Molverhältnisse von Verbindung IV zu Rh(CO)₂acac (0,388 mmol) eingestellt wurden.

**Tabelle 2**

| Bsp. Nr. | IV/Rh | Umsatz % | Selektivität, mol-% | | | | | | 5-FVSE |
|---|---|---|---|---|---|---|---|---|---|
| | | | 4-PSE | 2-PSE | VSE | 5-FVSE | 4-FVSE | 3-FVSE | FVSE |
| 3 (aus Tab. 1) | 4^{a} | 84,7 | 0,7 | 13,1 | 6,4 | 68,4 | 7,4 | 4,1 | 86 |
| 10 | 8 | 79,5 | 1,3 | 7,7 | 3,6 | 73,4 | 7,9 | 6,1 | 84 |
| 11 | 12 | 78,8 | 1,0 | 7,0 | 3,3 | 74,9 | 7,8 | 6,1 | 84 |
| 12 | 20 | 81,8 | 1,5 | 6,1 | 3,5 | 74,8 | 7,9 | 6,1 | 84 |

| | | | | | | | | | |
|---|---|---|---|---|---|---|---|---|---|
| ^{a}) 4 bar Überdruck; bei den übrigen Versuchen 5 bar | | | | | | | | | |

Man erkennt, daß durch die Steigerung des Ligand/Rhodiumverhältnisses von 4:1 auf 20:1 die Selektivität zu 5-Formylvaleriansäuremethylester nur noch geringfügig erhöht wird.

Der Einfluß des Lösungsmittels kann den Beispielen 1 und 13 sowie 14 entnommen werden (Tabelle 3).

Beispiel 1 wurde wiederholt, wobei abweichend die in Tabelle 3 genannten Lösungmittel eingesetzt wurden.

**Tabelle 3**

| Bsp. Nr. | Lösungsmittel | Umsatz % | Selektivität, mol-% | | | | | | 5-FVSE |
|---|---|---|---|---|---|---|---|---|---|
| | | | 4-PSE | 2-PSE | VSE | 5-FVSE | 4-FVSE | 3-FVSE | FVSE |
| 13 | - | 73,1 | 1,8 | 18,1 | 5,7 | 52,7 | 14,7 | 7,1 | 71 |
| 1 | Toluol | 95,5 | 0,2 | 4,4 | 4,8 | 76,7 | 7,8 | 6,1 | 85 |
| 14 | Palatinol-AH | 79,0 | 1,3 | 14,4 | 9,5 | 61,4 | 9,5 | 4,0 | 82 |

Man erkennt, daß im Prinzip auch ohne Lösungsmittel gearbeitet werden kann, in Toluol jedoch eine bessere Selektivität an 5-Formylvaleriansäuremethylester erhalten wird. Auch hochsiedende Lösungsmittel wie Bis-(2-ethylhexyl)-phthalat sind geeignet.

### Beispiel 15

Auch Mischungen isomerer Pentensäuremethylester können in die Reaktion eingesetzt werden, wie das Beispiel 15 zeigt:

Beispiel 1 wurde wiederholt, wobei jedoch anstelle des reinen 3-Pentensäuremethylesters folgendes Gemisch eingesetzt wurde:

| | | |
|---|---|---|
| 4-Pentensäuremethylester | 392 mMol | 44,8 mol-% |
| 3-Pentensäuremethylester | 329 mMol | 37,6 mol-% |
| 2-Pentensäuremethylester | 44 mMol | 5,0 mol-% |
| Valeriansäuremethylester | 110 mMol | 12,6 mol-% |

Das Reaktionsprodukt wies folgende Zusammensetzung auf:

| | |
|---|---|
| 4-Pentensäuremethylester | 1 mMol |
| 3-Pentensäuremethylester | 45 mMol |
| 2-Pentensäuremethylester | 84 mMol |
| Valeriansäuremethylester | 158 mMol |
| 5-Formylvaleriansäuremethylester | 512 mMol |
| 4-Formylvaleriansäuremethylester | 50 mMol |
| 3-Formylvaleriansäuremethylester | 22 mMol |

Bezogen auf eingesetztes Pentensäureestergemisch entspricht dies einem Gesamtumsatz von 89,5 %. Es errechnen sich folgende Selektivitäten:

| | |
|---|---|
| 2-Pentensäuremethylester | 7,8 mol-% |
| Valeriansäuremethylester | 6,9 mol-% |
| 5-Formylvaleriansäuremethylester | 74,8 mol-% |
| 4-Formylvaleriansäuremethylester | 7,3 mol-% |
| 3-Formylvaleriansäuremethylester | 3,2 mol-% |

Das Verhältnis 5-Formylvaleriansäuremethylester zu 3-, 4- und 5-Formylvaleriansäuremethylester (n-Anteil) beträgt 88 %.

### Beispiel 16

Auch 2-Pentensäuremethylester kann in die Reaktion eingesetzt werden, wie das Beispiel 16 zeigt:

Beispiel 1 wurde wiederholt, wobei abweichend statt 3-Pentensäuremethylester 2-trans-Pentensäuremethylester eingesetzt wurde. Es wurden folgende Ergebnisse erhalten:

| | |
|---|---|
| Umsatz: | 22,1 mol-% |

| Selektivität | |
|---|---|
| 4-Pentensäuremethylester | 0,4 mol-% |
| 3-Pentensäuremethylester | 13,6 mol-% |
| 2-cis-Pentensäuremethylester | 11,6 mol-% |
| Valeriansäuremethylester | 62,8 mol-% |
| 5-Formylvaleriansäuremethylester | 7,2 mol-% |
| 4-Formylvaleriansäuremethylester | 1,0 mol-% |
| 3-Formylvaleriansäuremethylester | 1,3 mol-% |
| 2-Formylvaleriansäuremethylester | 1,9 mol-% |

Man erkennt, daß der 2-trans-Pentensäuremethylesterumsatz nur gering ist und überwiegend das Hydrierprodukt Valeriansäuremethylester ensteht. Bei den Formylvaleriansäuremethylestern wird allerdings ein n-Anteil von 62 % beobachtet. Mit konventionellem Rhodium/Triphenylphosphinkatalysator wird hier nahezu ausschließlich 2-Formylvaleriansäuremethylester in hoher Ausbeute (Druck 280 bar) erhalten.

### Beispiel 17

In einem 1 l Hubrührautoklaven werden 100 g 4-Pentensäuremethylester in 300 g Toluol als Lösungsmittel sowie 1,486 g Polyphosphit der Formel IV (1,5 mmol) und 40 mg (0,388 mmol) Rhodium in Form der Komplexverbindung Rh(CO)₂-acetylacetonat vorgelegt. Der aktive Katalysator bildet sich unter Reaktionsbedingungen. Das Reaktionsgemisch wird auf 70°C aufgeheizt und dann mit einem Gemisch aus 50 Vol.-% Kohlenmonoxid und 50 Vol.-% Wasserstoff auf einen Druck von 4 bar eingestellt. Wenn während der Reaktion der Druck im Reaktor unter 2 bar fällt, wird durch Nachpressen des genannten Gasgemisches der Druck wiederum auf 4 bar erhöht. Nach einer Reaktionszeit von 1,5 h wird das Reaktionsgemisch abgekühlt, entspannt und analysiert.

| | |
|---|---|
| Umsatz an 4-Pentensäuremethylester: | 90,5 mol-% |

| Selektivität | |
|---|---|
| Valeriansäuremethylester | 0,4 mol-% |
| 5-Formylvaleriansäuremethylester | 92,9 mol-% |
| 4-Formylvaleriansäuremethylester | 5,9 mol-% |
| andere Nebenprodukte | 0,8 mol-% |

Das Verhaltnis 5-Formylvaleriansäuremethylester zu 4-Formylvaleriansäuremethylester beträgt 94:6.

### Beispiel 18

In einem 1 l-Hubrührautoklaven werden 100 g 3-Pentensäuremethylester in 300 g Toluol als Lösungsmittel der Hydroformylierung unter worfen. Das Reaktionsgemisch enthält als Katalysator 4,71 g der Verbindung XXXI (2,33 mmol) und 40 mg (0,388 mmol) Rhodium in Form der Komplexverbindung Rh(CO)₂acac (acac = Acetylacetonat). Das Reaktionsgemisch wird auf 100°C aufgeheizt und dann mit einem Gemisch aus 50 Vol.-% CO und 50 Vol.-% H₂ ein Druck von 5 bar eingestellt. Während der Reaktion wird der Druck im Reaktor durch Nachpressen eines Gasgemisches gleicher Zusammensetzung über einen Druckregler auf 5 bar gehalten. Nach einer Reaktionszeit von 5 Stunden ergibt die Analyse des Reaktionsgemisches folgende Umwandlung des 3-Pentensäuremethylester (in Mol %):

| | |
|---|---|
| Umsatz: | 72,1 mol-% |

| Selektivität | |
|---|---|
| 4-Pentensäuremethylester | 0,7 mol-% |
| 2-cis-Pentensäuremethylester | 0,2 mol-% |
| 2-trans-Pentensäuremethylester | 9,2 mol-% |
| Valeriansäuremethylester | 6,3 mol-% |
| 5-Formylvaleriansäuremethylester | 71,9 mol-% |
| 4-Formylvaleriansäuremethylester | 7,7 mol-% |
| 3-Formylvaleriansäuremethylester | 4,0 mol-% |

Die Ausbeute an Wertprodukt 5-Formylvaleriansäuremethylester beträgt 51,8 % und das Verhältnis 5-Formylvaleriansäuremethylester zu 3- und 4-Formylvaleriansäuremethylester beträgt 86:14.

### Beispiel 19

In einem 1 l-Hubrührautoklaven werden 100 g 3-Pentensäuremethylester in 300 g Toluol als Lösungsmittel der Hydroformylierung unterworfen. Das Reaktionsgemisch enthält als Katalysator 1,49 g der Verbindung IV (1,684 mmol) und 40 mg (0,388 mmol) Rhodium in Form der Komplexverbindung Rh(CO)₂acac (acac = Acetylacetonat). Das Reaktionsgemisch wird auf 100°C aufgeheizt und dann mit einem Gemisch aus 50 Vol.-% CO und 50 Vol.-% H₂ ein Druck von 4 bar eingestellt. Während der Reaktion wird der Druck im Reaktor durch Nachpressen eines Gasgemisches gleicher Zusammensetzung über einen Druckregler auf 4 bar gehalten. Nach einer Reaktionszeit von 5 Stunden ergibt die Analyse des Reaktionsgemisches folgende Umwandlung des 3-Pentensäuremethylester (in Mol %):

| | |
|---|---|
| Umsatz: | 49,3 mol-% |

| Selektivität | |
|---|---|
| 4-Pentensäuremethylester | 6,8 mol-% |
| 2-cis-Pentensäuremethylester | 2,1 mol-% |
| 2-trans-Pentensäuremethylester | 47,7 mol-% |
| Valeriansäuremethylester | 2,6 mol-% |
| 5-Formylvaleriansäuremethylester | 26,2 mol-% |
| 4-Formylvaleriansäuremethylester | 9,5 mol-% |
| 3-Formylvaleriansäuremethylester | 5,2 mol-% |

Die Ausbeute an Wertprodukt 5-Formylvaleriansäuremethylester beträgt 12,9 % und das Verhältnis 5-Formylvaleriansäuremethylester zu 3- und 4-Formylvaleriansäuremethylester beträgt 64:36.

### Vergleichsbeispiel 2

In einem 1 l-Hubrührautoklaven werden 100 g 3-Pentensäuremethylester in 300 g Toluol als Lösungsmittel der Hydroformylierung unterworfen. Das Reaktionsgemisch enthält als Katalysator 6,10 g Triphenylphosphit (19,3 mmol) und 40 mg (0,388 mmol) Rhodium in Form der Komplexverbindung Rh(CO)₂acac (acac = Acetylacetonat). Das Reaktionsgemisch wird auf 100°C aufgeheizt und dann mit einem Gemisch aus 50 Vol.-% CO und 50 Vol.-% H₂ ein Druck von 4 bar eingestellt. Während der Reaktion wird der Druck im Reaktor durch Nachpressen eines Gasgemisches gleicher Zusammensetzung über einen Druckregler auf 4 bar gehalten. Nach einer Reaktionszeit von 5 Stunden ergibt die Analyse des Reaktionsgemisches folgende Umwandlung des 3-Pentensäuremethylester (in Mol %):

| | |
|---|---|
| Umsatz: | 86,9 mol-% |

| Selektivität | |
|---|---|
| 4-Pentensäuremethylester | 0,4 mol-% |
| 2-cis-Pentensäuremethylester | 0,3 mol-% |
| 2-trans-Pentensäuremethylester | 7,9 mol-% |
| Valeriansäuremethylester | 8,4 mol-% |
| 5-Formylvaleriansäuremethylester | 27,4 mol-% |
| 4-Formylvaleriansäuremethylester | 27,5 mol-% |
| 3-Formylvaleriansäuremethylester | 28,1 mol-% |

Die Ausbeute an Wertprodukt 5-Formylvaleriansäuremethylester beträgt 23,8 % und das Verhältnis 5-Formylvaleriansäuremethylester zu 3- und 4-Formylvaleriansäuremethylester beträgt 33:67.

Man erkennt, daß nach dem Vergleichsbeispiel 2 zwar eine hohe Formylvalerianesterausbeute erhalten wird, der gewünschte 5-Formylvaleriansäuremethylester aber nur in untergeordnetem Maße entsteht.

## Patentansprüche

1. Verfahren zur Herstellung von ω-Formyl-C₄-C₂₀-Alkancarbonsäureestern durch Umsetzen von C₄-C₂₀-Alkencarbonsäureestern mit Kohlenmonoxid und Wasserstoff bei einer Temperatur von 30 bis 150°C in flüssiger Phase in Gegenwart von Rhodiumcarbonylkomplexen und in Gegenwart von mindestens einem tertiären organischem Polyphosphit mit 2 bis 6 Phosphoratomen im Molekül in dem jeweils eine Bindung an jedem Phosphoratom über eine Sauerstoffbrücke an einen substituierten oder nicht substituierten mindestens zweiwertigen Arylen- oder Bisarylenrest, einen Alkylenrest, der ein Sauerstoffatom in Kette enthalten kann, oder an einen Rest mit zwei isolierten Arylresten über die Arylreste gebunden ist und zwei Bindungen an jedem Phosphoratom über eine Sauerstoffbrücke an einen substituierten oder nicht substituierten zweiwertigen Arylen-, Bisarylenrest, einen Alkylenrest oder einen Rest mit zwei isolierten Arylresten über die Arylreste gebunden sind, oder zwei Bindungen an mindestens einem Phosphoratom jeweils getrennt über eine Sauerstoffbrücke jeweils an einen einwertigen substituierten oder nicht substituierten Aryl-, Bisaryl-, Alkyl- Aralkyl- oder Cycloalkylrest gebunden sind, dadurch gekennzeichnet, daß man als C₄-C₂₀-Alkencarbonsäureester
(a) 3-Pentensäureester oder
(b) Gemische aus 2-, 3- und 4-Pentensäureester oder
(c) Gemische aus 3-Pentensäure-C₁-C₄-Alkylester mit 4-Pentensäure-C₁-C₄-Alkylester oder
(d) 2-Pentensäuremethylester oder
(e) 2-Pentensäureethylester verwendet.

2. Verfahren nach Anspruch 1, dadurch gekennzeichnet, daß man als 3-Pentensäureester 3-Pentensäure-C₁-C₄-Alkylester einsetzt.

3. Verfahren nach Anspruch 1 oder 2, dadurch gekennzeichnet, daß man als C₄-C₂₀-Alkencarbonsäureester 3-Pentensäuremethylester einsetzt.

4. Verfahren nach Anspruch 1, dadurch gekennzeichnet, daß man ein Gemisch aus 4-, 3- und 2-Pentensäuremethylester einsetzt.

5. Verfahren nach den Ansprüchen 1 bis 4, dadurch gekennzeichnet, daß man ein molares Verhältnis Polyphosphit, berechnet als Äquivalent Phosphor, zu Rhodium von 1:1 bis 300:1 einhält.

6. Verfahren nach den Ansprüchen 1 bis 5, dadurch gekennzeichnet, daß man ein unter den Reaktionsbedingungen inertes Lösungsmittel mitverwendet.

7. Verfahren nach den Ansprüchen 1 bis 5, dadurch gekennzeichnet, daß man den jeweils als Endprodukt anfallenden ω-Formylalkancarbonsäureester als Lösungsmittel verwendet.

8. Verfahren nach den Ansprüchen 1 bis 6, dadurch gekennzeichnet, daß man die bei der Hydroformylierung anfallenden Hochsieder als Lösungsmittel verwendet.

9. Verfahren nach den Ansprüchen 1 bis 8, dadurch gekennzeichnet, daß man ein Polyphosphit der Formel IV 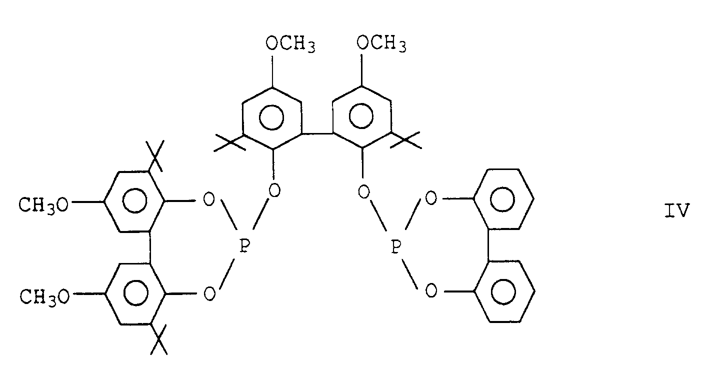 verwendet.

10. Verfahren nach Anspruch 1 bis 8, dadurch gekennzeichnet, daß man ein Polyphosphit der Formel XXXI 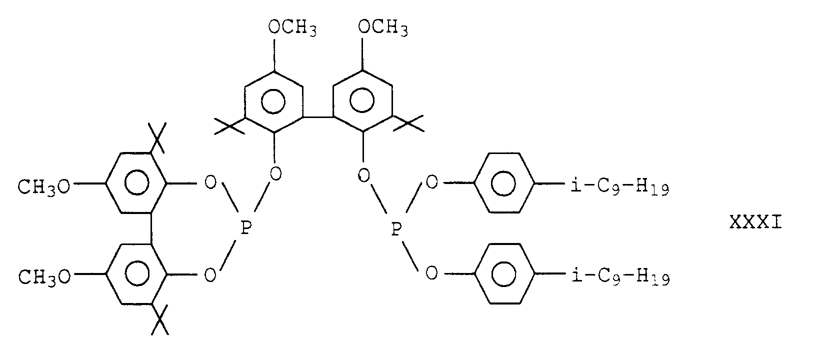 verwendet.

## Claims

1. A process for preparing ω-formyl-C₄-C₂₀-alkanecarboxylic esters by reacting C₄-C₂₀-alkenecarboxylic esters with carbon monoxide and hydrogen at from 30 to 150°C in liquid phase in the presence of rhodium carbonyl complexes and in the presence of at least one tertiary organic polyphosphite which has from 2 to 6 phosphorus atoms in the molecule and in which, in each case, one bond on each phosphorus atom is linked via an oxygen bridge to a substituted or unsubstituted at least divalent arylene or bisarylene radical to an alkylene radical which can contain an oxygen in the chain, or to a radical with two isolated aryl radicals via the aryl radicals, and two bonds on each phosphorus atom are linked via an oxygen bridge to a substituted or unsubstituted divalent arylene or bisarylene radical, to an alkylene radical or to a radical with two isolated aryl radicals via the aryl radicals, or two bonds on at least one phosphorus atom are, in each case, separately linked via an oxygen bridge in each case to a monovalent substituted or unsubstituted aryl, bisaryl, alkyl, aralkyl or cycloalkyl radical, wherein the C₄-C₂₀-alkenecarboxylic esters used are
(a) 3-pentenoic esters or
(b) mixtures of 2-, 3- and 4-pentenoic esters or
(c) mixtures of C₁-C₄-alkyl 3-pentenoate with C₁-C₄-alkyl 4-pentenoate or
(d) methyl 2-pentenoate or
(e) ethyl 2-pentenoate.

2. A process as claimed in claim 1, wherein C₁-C₄-alkyl 3-pentenoates are used as 3-pentenoic esters.

3. A process as claimed in claim 1 or 2, wherein methyl 3-pentenoate is used as C₄-C₂₀-alkenecarboxylic ester.

4. A process as claimed in claim 1, wherein a mixture of methyl 4-, 3- and 2-pentenoates is used.

5. A process as claimed in claims 1 to 4, wherein the molar ratio of polyphosphite, calculated as phosphorus equivalent, to rhodium is from 1:1 to 300:1.

6. A process as claimed in claims 1 to 5, wherein a solvent inert under the reaction conditions is also used.

7. A process as claimed in claims 1 to 5, wherein the ω-formylalkanecarboxylic ester which is the final product in each case is used as solvent.

8. A process as claimed in claims 1 to 6, wherein the high boilers resulting from the hydroformylation are used as solvent.

9. A process as claimed in claims 1 to 8, wherein a polyphosphite of the formula IV 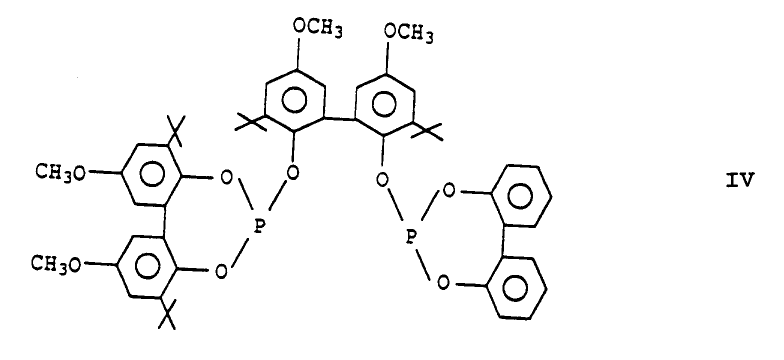 is used.

10. A process as claimed in claims 1 to 8, wherein a polyphosphite of the formula XXXI 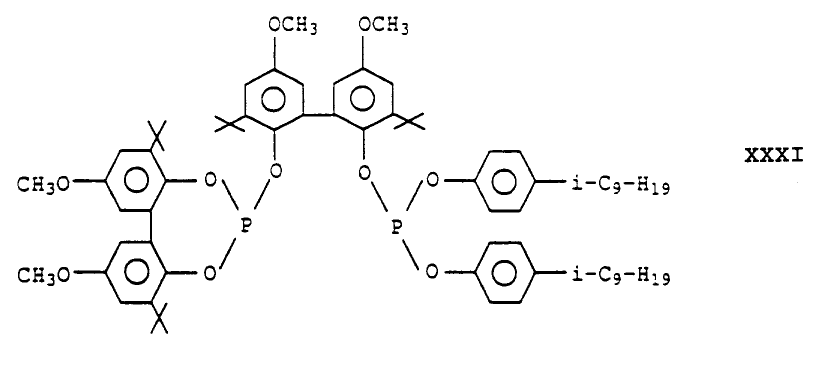 is used.

## Revendications

1. Procédé de préparation d'esters d'acides ω-formyl(alcane en C₄-C₂₀)carboxyliques par réaction d'esters d'acides (alcène en C₄-C₂₀)carboxyliques avec du monoxyde de carbone et de l'hydrogène à une température de 30 à 150°C, en phase liquide, en présence de complexes rhodium-carbonyle et en présence d'au moins un polyphosphite organique tertiaire à 2-6 atomes de phosphore dans la molécule, dans lequel une liaison sur chaque atome de phosphore est reliée chaque fois, par l'intermédiaire d'un pont oxygène, à un reste arylène ou bisarylène au moins bivalent, substitué ou non substitué, à un reste alkylène qui peut contenir un atome d'oxygène dans la chaîne ou, par les restes aryle, à un reste renfermant deux restes aryle séparés, et deux liaisons sur chaque atome de phosphore sont reliées, par l'intermédiaire d'un pont oxygène, à un reste arylène, bisarylène au moins bivalent, substitué ou non substitué, à un reste alkylène ou, par les restes aryle, à un reste renfermant deux restes aryle séparés, ou deux liaisons sur un atome de phosphore au moins sont reliées chacune séparément, par l'intermédiaire d'un pont oxygène, à un reste aryle, bisaryle, alkyle, aralkyle ou cycloalkyle monovalent substitué ou non substitué, caractérisé en ce que l'on utilise, comme ester d'acide (alcène en C₄-C₂₀)carboxylique,
a) des esters d'acide 3-penténoïque ou
b) des mélanges d'esters d'acides 2-, 3- et 4-penténoïques ou
c) des mélanges d'esters alkyliques en C₁-C₄ d'acide 3-penténoïque et d'esters alkyliques en C₁-C₄ d'acide 4-penténoïque ou
d) du 2-penténoate de méthyle ou
e) du 2-penténoate d'éthyle.

2. Procédé selon la revendication 1, caractérisé en ce que l'on utilise, comme esters d'acide 3-penténoïque, des esters alkyliques en C₁-C₄ d'acide 3-penténoïque.

3. Procédé selon la revendication 1 ou 2, caractérisé en ce que l'on utilise, comme ester d'acide (alcène en C₄-C₂₀)carboxylique, du 3-penténoate de méthyle.

4. Procédé selon la revendication 1, caractérisé en ce que l'on utilise un mélange de 4-, 3- et 2-penténoates de méthyle.

5. Procédé selon l'une quelconque des revendications 1 à 4, caractérisé en ce que l'on maintient un rapport molaire du polyphosphite, calculé en équivalent de phosphore, au rhodium de 1:1 à 300:1.

6. Procédé selon l'une quelconque des revendications 1 à 5, caractérisé en ce que l'on utilise simultanément un solvant inerte dans les conditions de la réaction.

7. Procédé selon l'une quelconque des revendications 1 à 5, caractérisé en ce que l'on utilise, comme solvant, l'ester d'acide ω-formylalcanecarboxylique qui est formé en tant que produit final.

8. Procédé selon l'une quelconque des revendications 1 à 6, caractérisé en ce que l'on utilise, comme solvant, les constituants de point d'ébullition élevé qui sont formés au cours de l'hydroformylation.

9. Procédé selon l'une quelconque des revendications 1 à 8, caractérisé en ce que l'on utilise un polyphosphite de formule IV

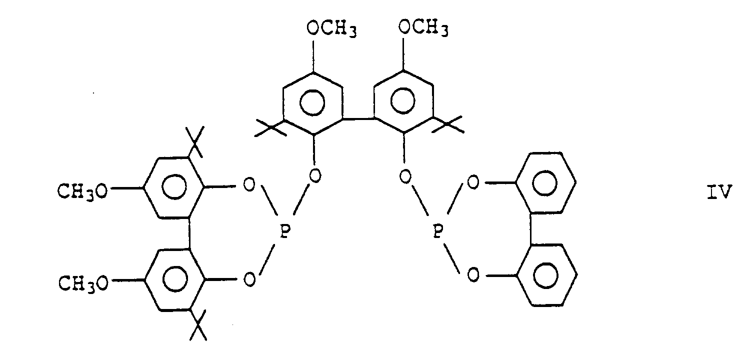

10. Procédé selon l'une quelconque des revendications 1 à 8, caractérisé en ce que l'on utilise un polyphosphite de formule XXXI
